# EUROPEAN PATENT APPLICATION

(11) **EP 1 650 209 A1**
(43) Date of publication of application: **26.04.2006**
(21) Application number: 04024779.3
(22) Date of filing: 18.10.2004
(51) Int. Cl.: C07D 493/10, C07D 493/20, A61K 31/352

(54) **Griseusin derivatives and their use as antitumor agents**

(71) Applicant: Oncotest GmbH, 79108 Freiburg (DE)
(72) Inventor: Grabley, Susanne Prof.Dr., 65779 Kelkheim (DE); Sattler, Isabel Dr., 07745 Jena (DE); Roemer, Ernst Dr., 07751 Jean (DE); Huang, Xueshi Dr., 07745 Jena (DE); He, Jian Dr., Los Angeles,CA 90095-1668 (US); Fiebig, Heinz-Herbert Prof.Dr., 79110 Freiburg (Lehen) (DE); Maier, Armin Dr., 79211 Denzlingen (DE); Kelter, Gerhard Dr., 79238 Ehrenkirchen (Kirchhofen) (DE)
(74) Representative: Müller-Boré & Partner Patentanwälte

(57) **Abstract**

The present invention relates to specific griseusin derivatives, pharmaceutical compositions comprising said griseusin derivatives and the use of these derivatives for the manufacture of a medicament for the treatment of cancers, particularly for the treatment of human solid cancers.

## Description

The present invention relates to specific griseusin derivatives, pharmaceutical compositions comprising said griseusin derivatives and the use of these derivatives for the manufacture of a medicament for the treatment of cancers, particularly for the treatment of human solid cancers.

Griseusin and its derivatives belong to the pyranonaphthoquinone family of antibiotics, such as kalfungin, arizonin or actinorhodin, which exhibit inhibitory activity against a variety of pathogenic fungi, yeasts and gram-positive bacteria as well as antiviral activity. Another property of these antibiotics is their ability to act as biore-ductive DNA alkylating agents via quinone methide intermediates thereby resulting in cross-linking of DNA strands. These alkylated DNA adducts then interfere with the cell replication process. A broad and strong antimicrobial activity against gram-positive bacteria and some gram-negative bacteria of 4'-deacetylgriseusin A and B, respectively, have been shown by Igarashi et al. (Igarashi, M. et al., *J. Antibiotics,* 1995, 48, 1502-1505).

However, griseusin derivatives have never been investigated with respect to the treatment of cancers, in particular, human solid tumors. Furthermore, there still remains a need for the provision of substances exhibiting remarkable effectiveness against cancer, in particular, human solid tumors.

Thus, the technical problem underlying the present invention is to provide new active substances which can be used in the treatment of cancers, in particular, in the treatment of human solid tumors and metastasis thereof.

The solution to the above technical problem is achieved by the embodiments characterized in the claims.

In particular, the present invention provides relates to griseusin derivatives represented by the following formula (I): 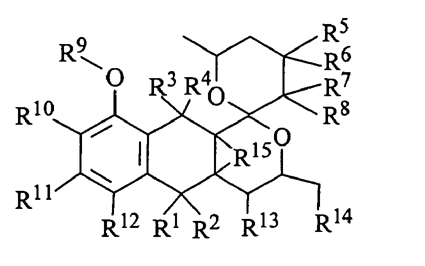 wherein R¹, R², R³ and R⁴ independently from each other represent hydrogen, hydroxy, a straight-chain or branched-chain alkyl group having 1 to 12 carbon atoms, a straight-chain or branched-chain alkoxy group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a substituted or unsubstituted aryloxy group which can comprise one or more heteroatoms, a (-C(O)-Rₐ) group or a (-C(S)-Rₐ) group, or at least one of R¹ and R² when taken together and R³ and R⁴ when taken together, respectively, stands for an oxo group (=O), thioxo group (=S), imino group (=NRₐ), cyanimino group (=NCN) or alkylidene group (=CRₐR_{b}),
with Rₐ and R_{b} independently from each other being selected from a group α, consisting of hydrogen, a straight-chain or branched-chain alkyl group having 1 to 12 carbon atoms, a cycloalkyl group having 3 to 10 carbon atoms and optionally comprising one or more heteroatoms, and a substituted or unsubstituted aryl group which can comprise one or more heteroatoms, a substituted or unsubstituted aralkyl group which can comprise one or more heteroatoms, or
at least one of R¹ and R² when taken together and R³ and R⁴ when taken together, respectively, forms together with the respective spiro carbon atom of ring B a 5 to 8 membered cyclic ketal structure and being optionally substituted by one or more substituents selected from group α, or
R², R⁴ and R¹⁵ are not present so that ring B is an aromatic ring substituted in its para-positions by R¹ and R³;
R⁵, R⁶, R⁷ and R⁸ independently from each other represent hydrogen, hydroxy, a straight-chain or branched-chain alkyl group having 1 to 12 carbon atoms, a straight-chain or branched-chain alkoxy group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a substituted or unsubstituted aryloxy group which can comprise one or more heteroatoms, a (-C(O)-Rₐ) group, a (-C(S)-Rₐ) group, a (-OC(O)-Rₐ) group, a (-OC(S)-Rₐ) group, a (-OC(O)-ORₐ) group or a (-(CH₂)ₙ-CRₐ=CRₐR_{b}) group wherein n is zero or an integer from 1 to 12 and with Rₐ and R_{b} being as defined above,or
at least one of R⁵ and R⁶ when taken together and R⁷ and R⁸ when taken together, respectively, stands for an oxo group (=O), thioxo group (=S), imino group (=NRₐ), cyanimino group (=NCN) or alkylidene group (=CRₐR_{b}) with Rₐ and R_{b} as defined above, or forms together with the respective spiro carbon atom a cyclic ketal structure having 5 to 8 carbon atoms and optionally substituted by one or more substituents selected from group α;
R⁹ represents hydrogen, a straight-chain or branched-chain alkyl group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a (-C(O)-Rₐ) group, a (-C(S)-Rₐ) group or a (-(CH₂)ₙ-CRₐ=CRₐR_{b}) group with n, Rₐ and R_{b} as defined above;
R¹⁰, R¹¹ and R¹² are independently glycosidic groups particularly of furanoic or pyranoic structure and optionally being deoxygenated or aza-substituted;
R¹³ represents hydrogen, hydroxy, a straight-chain or branched-chain alkoxy group having 1 to 12 carbon atoms, a (-C(O)-Rₐ) group, a (-C(S)-Rₐ) group, a (-OC(O)-Rₐ) group or a (-OC(S)-Rₐ) group, with Rₐ as defined above,
R¹⁴ represents hydrogen, hydroxy, a straight-chain or branched-chain alkyl group having 1 to 12 carbon atoms, a straight-chain or branched-chain alkoxy group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a substituted or unsubstituted aryloxy group which can comprise one or more heteroatoms, a (-C(O)-Rₐ) group, a (-C(S)-Rₐ) group, a (-OC(O)-Rₐ) group, a (-OC(S)-Rₐ) group or a (-COORₐ) group with Rₐ as defined above;
or R¹³ and R¹⁴ when taken together form the following lacton structure or lactol structure, respectively, 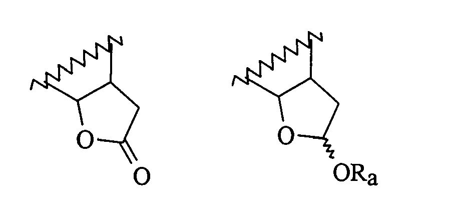 with Rₐ as defined above, and
R¹⁵ represents an alkylene group (-C(Rₐ)(R_{b})-), a (-CH₂-N=N-) group, an oxygen atom or a sulfur atom with Rₐ and R_{b} as defined above; or
R¹⁵ is not present, but stands for a double bond together with the adjacent ring carbon atoms in ring B to form a quinoid or semi-quinoid system in said ring B;
with the proviso that the griseusin derivative is not
- spiro[1H-naphtho[2,3-c]pyran-1,2'-[2H]pyran]-3-acetic acid, (3,3',4,4',5,5',6',10-octahydro-3',4',9-trihydroxy-6'-methyl-5,10-dioxo-) (deacetylgriseusin B),
- spiro[1H-naphtho[2,3-c]pyran-1,2'-[2H]pyran]-3-acetic acid, (4'-(acetyloxy)-3,3',4,4',5,5',6',10-octahydro-3',9-dihydroxy-6'-methyl-5,10-dioxo-) (griseusin B),
- spiro[1H-naphtho[2,3-c]pyran-1,2'-[2H]pyran]-3-acetic acid, (3',4'-bis(acetyloxy)-3,3',4,4',5,5',6',10-octahydro-9-hydroxy-6'-methyl-5,10-dioxo-),
- spiro[5H-furo[3,2-b]naphtho[2,3-d]pyran-5,2'-[2H]pyran]-2,6,11(3H)-trione, (3',3a,4',5',6',11b-hexahydro-3',4',7-trihydroxy-6'-methyl-) (deacetylgriseusin A),
- spiro[5H-furo[3,2-b]naphthol[2,3-d]pyran-5,2'-[2H]pyran]-2,6,11(3H)-trione, (4'-(acetyloxy)-3',3a,4',5',6',11b-hexahydro-3',7-dihydroxy-6'-methyl-) (griseusin A),
- spiro[5H-furo[3,2-b]naphtho[2,3-d]pyran-5,2'-[2H]pyran]-2,6,11(3H)-trione,3'4'-bis(acetyloxy)-3',3a,4',5',6',11b-hexahydro-7-hydroxy-6'-methyl-,
- spiro[1H-naphtho[2,3-c]pyran-1,2'-[2H]pyran]-3-acetic acid, 4'-(acetyloxy)-3,3',4,4',5,5',6',10-octahydro-3'hydroxy-9-methoxy-6'-methyl-5,10-dioxo-,
- spiro[5H-furo[3,2-b]naphtho[2.3-d]pyran-5,2'-[2H]pyran]-2,6,11(3H)-trione, 4'-(acetyloxy)-3',3a,4',5',6',11b-hexahydro-7-methoxy-6'-methyl-3'-(phenylmethoxy)-,
- spiro[5H-furo[3,2-b]naphtho[2,3-d]pyran-5,2'-[2H]pyran]2,6,11(3H)-trione, 3',4',7-tris(acetyloxy)-3',3a,4',5',6',11b-hexahydro-6'-methyl-
- carbonic acid, 4'-(acetyloxy)-2,3,3',3a,4',5',6,6',11,11b-decahydro-7-hydroxy-6'-methyl-2,6,11-trioxospiro[5H-furo[3,2-b]naphtho[2,3-d]pyran-5,2'-[2H]pyran]-3'-yl methyl ester, and
- carbonic acid, 4'-(acetyloxy)-2,3,3',3a,4',5',6,6',11,11b-decahydro-6'methyl-2,6,11-trioxospiro[5H-furo[3,2-b]naphtho[2,3-d]pyran-5,2'-[2H]pyran]-3',7-diyl dimethyl ester.

As may be understood from the above mentioned exceptions the following combinations of the groups R¹ to R¹⁴ in the griseusin derivatives of the formula (I) are excluded from the present invention:

| | R¹/R² and R³/R⁴ | R⁵ | R⁶ | R⁷ | R⁸ | R⁹ | R¹⁰, R¹¹, R¹² | R¹³ | R¹⁴ |
|---|---|---|---|---|---|---|---|---|---|
| 1 | Carbonyl | OH | H | OH | H | H | H | H | C(O)OH |
| 2 | Carbonyl | OC(O)CH₃ | H | OH | H | H | H | H | C(O)OH |
| 3 | Carbonyl | OC(O)CH₃ | H | OC(O)CH₃ | H | H | H | H | C(O)OH |
| 4 | Carbonyl | OH | H | OH | H | H | H | lacton | |
| 5 | Carbonyl | OC(O)CH₃ | H | OH | H | H | H | lacton | |
| 6 | Carbonyl | OC(O)CH₃ | H | OC(O)CH₃ | H | H | H | lacton | |
| 7 | Carbonyl | OC(O)CH₃ | H | OH | H | CH₃ | H | H | C(O)OH |
| 8 | Carbonyl | OC(O)CH₃ | H | OBn | H | CH₃ | H | lacton | |
| 9 | Carbonyl | OC(O)CH₃ | H | OC(O)CH₃ | H | OC(O)CH₃ | H | lacton | |
| 10 | Carbonyl | OC(O)OCH₃ | H | OC(O)CH₃ | H | H | H | | lacton |
| 11 | Carbonyl | OC(O)OCH₃ | H | OC(O)CH₃ | H | OC(O)OCH₃ | H | | lacton |

In a preferred embodiment of the present invention the groups R¹ to R¹⁵ of the griseusin derivative of the formula (I) are defined as follows:
R¹, R², R³ and R⁴ independently from each other represent hydrogen, hydroxy, a straight-chain or branched-chain alkoxy group having 1 to 12 carbon atoms or R¹/R² and/or R³/R⁴ stand(s) for an oxo group (=O) or form(s) together with the respective spiro carbon atom of ring B a cyclic ketal structure having 5 to 8 carbon atoms and optionally substituted by one or more substituents selected from group α;
R⁵, R⁶, R⁷ and R⁸ independently from each other represent hydrogen, hydroxy, a straight-chain or branched-chain alkyl group having 1 to 12 carbon atoms, a straight-chain or branched-chain alkoxy group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a substituted or unsubstituted aryloxy group which can comprise one or more heteroatoms, a (-C(O)-Rₐ) group, a (-C(S)-Rₐ) group, a (-OC(O)-Rₐ) group, a (-OC(S)-Rₐ) group, a (-OC(O)-ORₐ) group or a (-(CH₂)ₙ-CRₐ=CRₐR_{b}) group with n, Rₐ and R_{b} being as defined above, or R⁵/R⁶ and/or R⁷/R⁸ stand(s) for an oxo group (=O) or form(s) together with the respective spiro carbon atom of ring B a 5 to 8 membered cyclic ketal structure being optionally substituted by one or more substituents selected from group α,
R⁹ represents hydrogen, a straight-chain or branched-chain alkyl group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a (-C(O)-Rₐ) group or a (-C(S)-Rₐ) group with Rₐ as defined above;
R¹³ represents hydrogen, hydroxy, a straight-chain or branched-chain alkyl group having 1 to 12 carbon atoms, a straight-chain or branched-chain alkoxy group having 1 to 12 carbon atoms or a (-C(O)-Rₐ) group,
R¹⁴ represents hydroxy, a straight-chain or branched-chain alkoxy group having 1 to 12 carbon atoms, a (-OC(O)-Rₐ) group, a (-OC(S)-Rₐ) group or a (-COORₐ) group with Rₐ as defined above,
or R¹³ and R¹⁴ when taken together form said lacton structure 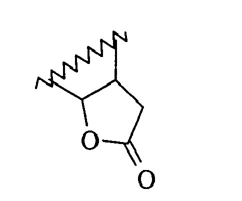 and
R¹⁵ represents an oxygen atom or is not present, but stands for a double bond together with the adjacent ring carbon atoms in ring B.

In a more preferred embodiment of the present invention the groups R¹ to R¹⁵ of the griseusin derivate of the formula (I) are defined as follows:
the groups R¹/R² and R³/R⁴ each form a carbonyl group;
at least one of R⁵/R⁶ and R⁷/R⁸ forms a carbonyl group, while the remaining R⁵ and R⁶ or R⁷ and R⁸, respectively, independently from each other represent hydrogen, hydroxy, a (-OC(O)-Rₐ) group or a (-OC(S)-Rₐ) group with Rₐ as defined above, or both of R⁵/R⁶ and R⁷/R⁸ form a carbonyl group;
R⁹ represents hydrogen or a straight-chain or branched-chain alkyl group having 1 to 12 carbon atoms;
R¹³ represents hydroxy and R¹⁴ represents a (-COORₐ) group with Rₐ as defined above or R¹³ and R¹⁴ when taken together form said lacton structure 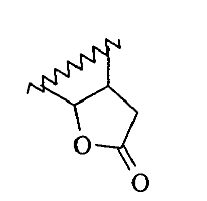 and
R¹⁵ is not present, but stands for a double bond together with the adjacent ring carbon atoms in ring B.

With respect to the term "a straight-chain or branched-chain alkoxy group having 1 to 12 carbon atoms", this should also include those alkoxy groups having one or more further ether bridges, like e.g. -O-(CH₂)ₘ-O-Rₐ or -O-(CH₂)ₘ-O-(CH₂)ₘ-O-Rₐ with m being independently from each other an integer from 1 to 4 and Rₐ as defined hereinabove.

With respect to the term "a straight-chain or branched-chain alkyl group having 1 to 12 carbon atoms", this should also include those alkyl groups having one or more ether bridges inserted, like e.g. -(CH₂)ₘ-O-Rₐ or -(CH₂)ₘ-O-(CH₂)ₘ-O-Rₐ with m being independently from each other an integer from 1 to 4 and Rₐ as defined hereinabove.

With respect to the (-(CH₂)ₙ-CRₐ=CRₐR_{b}) group as given hereinabove, in the course of the present invention, it is also be encompassed that there are more than one olefinic double bond within said alkenylene residue.

R¹⁰, R¹¹ and R¹² are independently glycosidic groups particularly of furanoic or pyranoic structure and optionally being deoxygenated or aza-substituted. Examples of such glycosidic groups are fructofuranoside, glucofuranoside, glucoside, fructoside, galactoside, or aminoglycoside groups bound *via* a glycosidic linkage to the griseusin moiety. Further, there are included nucleosides and nucleotides based on respective ribonucleic acids or desoxyribonucleic acids.

The above griseusin derivatives of the present invention can also be in the form of their isomers, stereoisomers or diastereomers. If the griseusin derivatives are chiral, the present invention may cover racemates, mixtures of the stereoisomers in varying ratios as well as the chiral compounds as such.

Further, the present griseusin derivatives comprise all stereoisomers, such as those which may exist due to asymmetric carbons on the various substituents, including enantiomeric forms (which may exist even in the absence of asymmetric carbons) and diastereomeric forms. Individual stereoisomers of the griseusin derivatives of the present invention may, for example, be substantially free of other isomers, or may be admixed, for example, as racemates or with all other, or other selected, stereoisomers.

To the extent that griseusin derivatives of the present invention and salts thereof may exist in their tautomeric form, all such tautomeric forms are contemplated herein as part of the present invention.

Preferably, the griseusin derivatives of the present invention may be in the form of salts. The term "salt(s)", as employed herein, denotes acidic and/or basic salts formed with inorganic and/or organic acids and bases. In addition, when a griseusin derivative of the invention contains both a basic moiety and an acidic moiety, "inner salts" may be formed and are included within the term "salt(s)" as used herein. Pharmaceutically acceptable (i.e., non-toxic, physiologically acceptable) salts are preferred, although other salts are also useful, e.g., in isolation or purification steps which may be employed during preparation. Salts of the griseusin derivatives of the invention may be formed, for example, by reacting a griseusin derivative of the invention with an amount of acid or base, such as an equivalent amount, in a medium such as one in which the salt precipitates or in an aqueous medium followed by lyophilization. Salts of the griseusin derivatives of the invention may also be formed by reacting a griseusin derivative of the present invention with an alkylating agent, for example, by quarternization of an amine.

The griseusin derivatives of the present invention which contain a basic moiety, may form salts with a variety of organic and inorganic acids. Exemplary acid addition salts include acetates, adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, 2 hydroxyethanesulfonates, lactates, maleates, methanesulfonates, 2-naphtalenesulfonates, nicotinates, nitrates, oxalates, pectinates, persulfates, 3- phenylpropionates, phosphates, picrates, pivalates, propionates, salicylates, succinates, sulfates, sulfonates, tartrates, thiocyanates, toluenesulfonates, such as tosylates, undecanoates, and the like.

The griseusin derivatives of the present invention which contain an acidic moiety may form salts with a variety of organic and inorganic bases. Exemplary basic salts include ammonium salts, alkali metal salts such as sodium, lithium, and potassium salts, alkaline earth metal salts such as calcium and magnesium salts, salts with organic bases (for example, organic amines) such as benzathines, dicyclohexylamines, N-methyl-D-glucamines, N-methyl-D-glucamides, t-butyl amines, and salts with amino acids such as arginine, lysine and the like.

Further, the griseusin derivatives of the present invention may be in the form of their solvates which are preferably hydrates of these derivatives.

Moreover, the griseusin derivatives of the present invention may be in the form of a prodrug. The term "prodrug", as employed herein, denotes a compound which, upon administration to a subject, undergoes chemical conversion by metabolic or chemical processes to yield a compound of the present invention, or a salt and/or solvate thereof.

Further, the present invention relates to pharmaceutical compositions comprising at least one griseusin derivative of the present invention as an active ingredient. These pharmaceutical compositions are particularly useful in cytotoxic/anticancer applications. They may, furthermore, comprise immunomodulating and/or immunosuppressive activities.

These pharmaceutical compositions may be made by using a griseusin derivative of the present invention, optionally in combination with a pharmaceutically acceptable carrier, excipient and/or diluent.

Examples of suitable pharmaceutical carriers, excipients and/or diluents are well known in the art and include phosphate buffered saline solutions, water, emulsions, such as oil/water emulsions, various types of wetting agents, sterile solutions etc.. Pharmaceutical compositions comprising such carriers can be formulated by well known conventional methods.

Aqueous carriers include water, alcoholic/aqueous solutions, emulsions and suspensions, including saline and buffered media. Parenteral vehicles include sodium chloride solution, Ringer's dextrose, dextrose and sodium chloride, lactated Ringer's and fixed oils. Intravenous vehicles include fluid and nutrient replenishers, electrolyte replenishers (such as those based on Ringer's dextrose), and the like. Preservatives and other additives may also be present in the pharmaceutical composition of the present invention such as antimicrobials, anti-oxidants, chelating agents, and inert gases. Furthermore, the pharmaceutical compositions of the present invention may comprise further agents depending on the intended use.

The pharmaceutical compositions according to the present invention can be prepared in various forms, such as granules, tablets, pills, suppositories, capsules, suspensions, salves, lotions and the like. Pharmaceutical grade organic or inorganic carriers and/or diluents suitable for oral and topical use can be used to formulate compositions containing the therapeutically-active compounds. Diluents known in the art include aqueous media, vegetable and animal oils and fats. Stabilizing agents, wetting and emulsifying agents, salts for varying the osmotic pressure or buffers for securing an adequate pH value, and skin penetration enhancers can be used as auxiliary agents. The pharmaceutical compositions may also include one or more of the following: carrier proteins such as serum albumin; buffers; fillers such as microcrystalline cellulose, lactose, corn and other starches; binding agents; sweeteners and other flavoring agents; coloring agents; and polyethylene glycol. Those additives are well known in the art, and are used in a variety of formulations.

Where desired, the griseusin derivatives of the present invention may be used in combination with one or more other types of therapeutic agents which may be administered of the same type (e.g. orally) in the same dosage form (e.g. orally), in a separate dosage form or by a different application form (e.g. by injection).

By "administered" herein is meant administration of a therapeutically effective dose of a griseusin derivative according to the present invention to a cell either in cell culture or in a patient. By "therapeutically effective dose" herein is meant a dose that produces the effects for which it is administered. The exact dose will depend on the purpose of the treatment, and will be ascertainable by one skilled in the art using known techniques. As is known in the art and described above, adjustments for systemic *versus* localized delivery, age, body weight, general health, sex, diet, time of administration, drug interaction and the severity of the condition may be necessary, and will be ascertainable with routine experimentation by those skilled in the art. Preferably, the griseusin derivatives of the present invention are administered to cells.

The pharmaceutical compositions can be administered to the subject at a suitable dose. Administration of the suitable compositions may be effected by different ways, e.g. by oral, rectal, intravenous, intraperitoneal, subcutaneous, intramuscular, topical, intradermal, intranasal or intrabronchial administration. It is particularly preferred that said administration is carried out by injection and/or delivery, e.g., to a site in an artery or directly into tissue. The pharmaceutical compositions of the present invention may also be administered directly to the target site, e.g., by biolistic delivery to an external or internal target site. The dosage regimen will be determined by the attending physician and clinical factors. As is well known in the medical arts, dosages for any patient depend upon many factors, including the severity of the condition, the patient's size, body surface area, age, the particular compound to be administered, sex, time and route of administration, general health, and other drugs being administered concurrently. Pharmaceutically active matter may be present in amounts from 1 ng to 10 mg/kg body weight per dose; however, doses below or above this exemplary range are envisioned, especially considering the aforementioned factors. If the regimen is a continuous infusion, it should for example also be in the range of 1 µg to 10 mg units per kilogram of body weight per minute.

A "patient" or "subject" for the purposes of the present invention includes both humans and other animals, particularly mammals, and other organisms. Thus, the griseusin derivatives of the present invention are applicable to both humans and animals. In the preferred embodiment the patient is a mammal, and in the most preferred embodiment the patient is human.

Typically, the griseusin derivatives of the present invention having pharmacological activity (e.g. cytotoxic activity) may be administered in a physiologically acceptable carrier to a patient, as described herein. Depending upon the manner of introduction, the griseusin derivatives may be formulated in a variety of ways as discussed below. The concentration of therapeutically active griseusin derivative in the formulation may vary from about 0.1-100 wt %. The agents may be administered alone or in combination with other treatments, i.e., radiation, or other chemotherapeutic agents.

The administration of the griseusin derivatives according to the present invention can be done in a variety of ways as discussed above, including, but not limited to, orally, subcutaneously, intravenously, intranasally, transdermally, intraperitoneally, intramuscularly, intrapulmonary, vaginally, rectally, or intraocularly.

In a preferred embodiment, the pharmaceutical compositions of the present invention are in a water soluble form, such as pharmaceutically acceptable salts, which is meant to include both acid and base addition salts.

Progress in the patient treated with one or more griseusin derivative(s) of the present invention can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate.

The griseusin derivatives of the present invention are particularly useful for the treatment of cancer, in particular, human solid tumors such as skin, breast, brain, uterus carcinomas, testicular carcinomas, etc. More particularly, the term "human solid tumors" according to the present invention preferably includes the following solid tumor diseases: sarcomas (angiosarcoma, fibrosarcoma, habdomyosarcoma, liposarcoma); lung: bronchogenic carcinoma (squamous cell, undifferentiated small cell, undifferentiated large cell, adenocarcinoma), alveolar (bronchiolar) carcinoma, mesothelioma; gastrointestinal: esophagus (squamous cell carcinoma, adenocarcinoma), stomach (carcinoma, leiomyosarcoma), pancreas (ductal adenocarcinoma), large bowel (adenocarcinoma, tubular adenoma, villous adenoma, hamartoma, leiomyoma); genitourinary tract: kidney (adenocarcinoma), bladder and urethra (squamous cell carcinoma, transitional cell carcinoma, adenocarcinoma), prostate (adenocarcinoma), testis (seminoma, teratoma, embryonal carcinoma, teratocarcinoma, choriocarcinoma); liver: hepatoma (hepatocellular carcinoma), cholangiocarcinoma, hepatoblastoma; bone: osteogenic sarcoma (osteosarcoma), fibrosarcoma, malignant fibrous histiocytoma), brain (astrocytoma, medulloblastoma, glioma, ependymoma, glioblastoma multiform, oligodendroglioma); gynecological: uterus (endometrial carcinoma), cervix (cervical carcinoma), ovaries (ovarian carcinoma [serous cystadenocarcinoma, mucinous cystadenocarcinoma, unclassified carcinoma], granulosa-thecal cell tumors; skin: malignant melanoma, basal cell carcinoma, squamous cell carcinoma. Thus, the term "cancerous cell" as provided herein, includes a cell afflicted by any one of the above identified conditions. The griseusin derivatives disclosed herein may also be useful in the treatment of benign proliferative disorders, like in the treatment of hypertrophies, hyperplasias as well as benign tumors, warts or condylomata.

The griseusin derivatives of the present invention can be prepared according to known chemical reactions or can be isolated from *Nocardiopsis sp.* strain HKI 0344 (DSM 16644). It is also possible to isolate precursors of the griseusin derivatives from *Nocardiopsis sp.* strain and to prepare derivatives thereof according to known chemical reactions. The griseusin derivatives can e.g. be isolated as described in the appended examples. The method for detection comprises thin layer chromatography (TLC) on silica gel with two solvent systems. Four staining reagents are used for the chemical profiling of metabolic patterns (Grabley S., Thiericke R., Drug discovery from Nature, Springer Verlag, Berlin). Those methanolic extracts with the most prominent and unique metabolic profiles are selected for further fractionation and purification. High pressure liquid chromatography (HPLC) on reversed phase silica gel (C18) with water/methanol-gradient as an elution solvent with UV as well as MS detection are used for the product analysis and production optimisation. It will be clear to those having ordinary skill in this art that the griseusin derivatives of the present invention can be synthesised according to standard methods which for example can be deduced from the following publication: March's Advanced Organic Chemistry: Reaction, Mechanisms and Structure, 5^{th} ed. by Michael B. Smith, Jerry March, Wiley-Interscience; 2001; Classics in Total Synthesis: Targets, Strategies, Methods by K. C. Nicolaou, E. J. Sorensen John VViley & Son Ltd, 1996 and The Art and Science of Total Synthesis at the Dawn of the Twenty-First Century. Nicolaou KC Vour-loumis, D, Winssinger N, Baran PS., Angew Chem Int Ed Engl 2000, 39 (1): 44 - 122.

The present invention is further explained by the following examples.

### Example 1: Isolation, Taxonomy and Cultivation of the Nocardiopsis sp. strain HKI 0344

The strain HKI 0344 of *Nocardiopsis sp.* was isolated from an alkaline soil sample (pH 9-10) collected in the region of Xinjiang, China, by using a standard dilution plating procedure and an alkaline isolation medium.

The strain HKI 0344 of *Nocardiopsis sp.* was identified on the basis of 16S rDNA analysis and is characterized by a well developed white to beige aerial mycelium on yeast extract-malt extract agar, a reddish brown to dark brown substrate mycelium and the formation of a brown soluble pigment. In old cultures hyphae completely fragment into spores. In liquid culture the characteristic zig-zag hyphae are observed. Further, the cell wall contains meso-2,6-diaminopimelic acid (meso-DAP).

The laboratory cultivation of this organism is performed on yeast extract - malt extract agar that consists of 0.4% glucose, 1.0% malt extract, 0.4% yeast extract and 2.0% agar or in liquid culture broth of the same composition without agar. After sterilization, the pH value of the medium is adjusted to pH 9.5-10.0 with sterilized NaOH (32%). The incubation temperature is 28°C. Stock cultures of strain HKl 0344 are maintained either in the vapour phase of liquid nitrogen by adding 5% DMSO to the yeast extract-malt extract medium or at -80°C by adding glycerol to the culture medium (1:1).

The *Nocardiopsis sp.* strain HKI 0344 has been deposited under the deposition number DSM 16644 at the Deutsche Sammlung von Mikroorganismen und Zellkul-turen GmbH (DSMZ), Braunschweig, Germany.

### Example 2: Isolation of the griseusin derivatives A to D

The compounds of the present invention can be obtained from the culture broth of the strain HKI 0344. Therefore, the strain HKI 0344 of *Nocardiopsis sp.* is cultivated under shaking conditions for 48h at 28°C in 500ml Erlenmeyer flasks containing 100ml of liquid yeast extract-malt extract medium with an initial pH value of pH 9.5-10.0. This culture is used as a seed culture for the inoculation of 100 ml fermentation medium (500ml Erlenmeyer flasks) consisting of 2% soy bean flour and 2% mannitol (pH 9.5-10.0) and is incubated under the same conditions (300 I, 6 days at 28°C on a soy bean flour medium).

After sufficient growth of the microorganism, the cultivation is terminated by separation of the cells. Briefly, the biomass and culture broth are separated using centrifugation or filtration. The compounds are then isolated from the culture filtrate. The initial work-up procedure involves adsorption on solid phase resin (e.g. Amberchrom 161c) and elution of the bound compounds with methanol (in accordance with Schmid, I. et al., Biomol. Screening, 1999, 4, p. 13-23). After evaporation of the organic solvent, the compounds are separated from unwanted material by standard chromatographic procedures as documented in the following. The chemical nature of all compounds, as shown below, are unequivocally determined by mass spectrometry and one- and two-dimensional ¹H- and ¹³C-NMR measurements (NMR spectra are taken on 300 MHz [¹H] and 75 MHz [¹³C] and are reported as: chemical shift [multiplicity (s = singlet, d = doublet, t = triplet, q = quartet)] in solution of CDCl₃. Solvents for the isolation procedure are free of flexibilizer or p.a. quality).

### Compound A (deacetylgriseusin A)

The steps of isolation and the data for structural assignment (MS, ¹H- and ¹³C-NMR) comply with Igarashi et al., J. Antibiotics, 1995, 48, p. 1502-1505.

### Compound B (4'-dehydro-deacetylariseusin A) (new compound according to the present invention)

This compound is isolated by column chromatography on sephadex LH 20, elution with methanol, and finally by column chromatography on silica gel (CHCI₃/MeOH) to obtain 36 mg of a red powder as the pure compound.

ESI-MS: m/z 438.2 [M + NH₄]⁺, 443.1 [M + Na]⁺, 862.8 [2M + Na]⁺; HREI-MS: *m*/*z* 402.0963 [M-H₂O]⁺ (calculated C₂₀H₁₈O₉, 402.0950); α_{D} (0.08, MeOH): -110;
¹H-NMR: 1.39 (d), 2.66 (m), 2.67 (d), 2.94 (dd), 3.25 (d), 4.21 (m), 4.68 (dd), 5.27 (d), 5.47 (d), 7.31 (dd), 7.66 (m), 11.9 (s);
¹³H-NMR: 21.4 (q), 35.9 (t), 47.5 (t), 66.6 (d), 68.1 (d), 69.4 (d), 75.9 (d), 99.5 (s), 115.2 (s), 119.6 (d), 125.5 (d), 131.2 (s), 137.1 (d), 138.4 (s), 140.5 (s), 162.2 (s), 173.1 (s), 181.6 (s), 187.3 (s), 203.0 (s).

### Compound C (4a,10a-Epoxy-deacetylgriseusin B) (new compound according to the present invention)

This compound is isolated by extraction with ethylacetate, repeated column chromatography on Sephadex LH 20 and finally elution with MeOH to obtain 25 mg of red powder as a pure compound.

ESI-MS: *m*/*z* 418.2 [M + NH₄]⁺, 423.1 [M + Na]⁺, 822.9 [2M + Na]⁺; HREI-MS: *m*/*z* 400.0821 [M]⁺ (calculated C₂₀H₁₆O₉, 400.0794); α_{D} (0.13, MeOH): +9.2;
¹H-NMR: 1.22 (d), 1.48 (m), 1.95 (m), 2.18 (dd), 2.5 (dd), 2.57 (d), 3.90 (m), 4.16 (m), 4.31 (m), 4.46 (d), 7.28 (d), 7.56 (d), 7.70 (dd);
¹³H-NMR: 21.2 (q), 28.9 (t), 40.5 (t), 42.5 (t), 63.9 (d), 64.8 (s), 65.5 (s), 67.0 (d), 69.1 (d), 75.3 (d), 98.7 (s), 116.2 (s), 119.9 (d), 125.8 (d), 137.2 (s), 138.2 (d), 163.8 (s), 174.7 (s), 190.6 (s), 194.8 (s).

### Compound D (deacetylgriseusin B)

The steps of isolation and the data for structural assignment (MS, ¹H- and ¹³C-NMR) comply with Igarashi et al., J. Antibiotics, 1995, 48, p. 1502-1505.

### Example 3: Syntheses of griseusin derivatives B and E to H (compounds B, E, F and G being new compounds according to the present invention)

The compounds B and E to H are synthesized by derivatisation of the isolated compounds A, B or C. Solvents used in these reaction processes are purchased in anhydrous quality if necessary, solvents for the purification are of p.a. quality. Reactions requiring an inert atmosphere are run under a blanket of dry argon.

### Compound B (4'-dehydro-deacetylgriseusin A)

To a solution of 200 mg (498 µmol) of compound A in 15 ml acetone are added 600 µl of freshly prepared Jones agent at room temperature, the addition is divided into three portions every hour. The mixture is stirred for two additional hours and is worked up by filtration through a short silica column. Then, compound B is purified by column chromatography on silica gel repeated twice and is eluated with a stepwise gradient from dichloromethane to dichloromethane/ethylacetate = 3/1, to obtain 160 mg (80%) of compound B. The data for structural assignment comply with the above obtained results of the isolated compound B.

### Compound E (4-hydroxy-deacetylgriseusin B methylester)

A solution of 50 mg (124 µmol) of compound C in 4 ml methanol is acidified with 40 µl of conc. hydrochloric acid and is allowed to stay at room temperature over night. After evaporation of the solvent the crude product is yielded nearly quantitatively. The purifiaction is conducted with a preparation plate chromatography on silica gel, first development dichloromethane/ethylacetate = 1/1, second development dichloromethane/ethylacetate = 1 : 2 to obtain 30 mg (56%) of compound E and some recovered starting material C (5 mg).

HPLC-ESI-MS: m/z 433.01 [M-H]⁻, 479.22 [M+CO₂-H]⁻, 866.93 [2M-H]⁻, 1300.95 [3M-H]⁻;
¹³C-NMR: 20.9 (q), 35.9 (t), 39.8 (t), 51.9 (d), 60.0 (d), 67.01 (d), 67.04 (d), 69.0 (d), 74.9 (d), 97.9 (s), 115.3 (s), 119.0 (d), 125.3 (d), 131.2 (s), 136.3 (d), 139.3 (s), 144.7 (s), 161.9 (s), 171.4 (s), 183.4 (s), 187.9 (s).

### Compound F (4'-dehydro-4-hydroxy-deacetylgriseusin B methylester)

A solution of 50 mg (125 µmol) of compound B in 20 ml methanol is acidified with 60 µl of conc. hydrochloric acid and is allowed to stay at room temperature over night. After evaporation of the solvent the crude product yields to 38 mg (70%). The purification is conducted with a preparation plate chromatography on silica gel, and the compound is eluted with dichloromethane/ethylacetate = 3/1, two developments.

HPLC-ESI-MS: m/z 433.01 [M+H]⁺, 454.96 [M+Na]⁺, 864.97 [2M+H]⁺, 886.99 [2M+Na]⁺;
¹H-NMR: 1.38 (d), 2.54 (d), 2.61 (m), 2.80 (m), 3.52 (d), 4.41 (m), 4.63 (m), 5.22 (d), 7.29 (dd), 7.61 (dd), 7.66 (dd), 12.0 (s);
¹³C-NMR: 21.5 (q), 34.7 (t), 47.5 (t), 51.9 (d), 59.8 (d), 67.8 (d), 68.8 (d), 76.0 (d), 100.0 (s), 115.3 (s), 119.4 (d), 125.4 (d), 131.4 (s), 136.7 (d), 137.0 (s), 145.1 (s), 162.1 (s), 171.1 (s), 182.7 (s), 188.1 (s), 203.7 (s).

### Compound G (4'-dehydro-4a,10a-epoxy-deacetylgriseusin B)

To a solution of 10 mg (23.8 µmol) of compound C in 2 ml acetone is added 29 µl of freshly prepared Jones agent at room temperature, the addition is divided into three portions every 30 min. After stirring of three additional hours the mixture is worked up by filtration through a short silica column. The purification is conducted on a column chromatography on silica gel and the compound is eluated stepwise with a gradient from dichloromethane to dichloromethane/ethylacetate = 3/1 to obtain 3 mg of compound G.

ESI-MS: *m*/*z* 419.5 [M+H]⁺, 436.5 [M+NH₄]⁺, 441.7 [M+Na]⁺, 859.6 [2M+Na]⁺.

### Compound H (griseusin A)

To a solution of 10 mg (24.8 µmol) of compound A in 1 ml dichloromethane are added a few cristals of DMAP, 10 µl TEA and 1.77 µl (1 eq.) of acetic acid chloride.

The mixture is allowed to stay at room temperature over night. Then water is added to the mixture which is extracted with dichloromethane, dried over sodium sulfate and the solvents are evaporated to quantitatively yield a crude product. The purification is carried out on a TLC plate, elution with dichloromethane/ethylacetate = 1/1 to obtain 5 mg (45%) of compound H. The data for structural assignment (MS, ¹H- and ¹³C-NMR) comply with Igarashi et al., 1995.

### Example 4: Tests for the Inhibition of the Growth of Various Human Cancer Cells and Human Cancer Xenografts

Using various human cancer cells *in vitro,* the concentrations of the test compounds (A, B, C, D, E, F), which inhibit the proliferation of cancer cells by 50% (IC₅₀) and 70% (IC₇₀) were determined. Specifically, each cell line of bladder cancer (BXF 1218L and T-24), glioblastoma (CNXF 498NL), colic cancer (HCT-116 and HT-29), gastric cancer (GXF 251 L), head and neck cancer (HNXF 536L), non small cell lung cancer (LXF 1121L, LXF 289L, LXF 526L, LXF 529L, LXF 629L and H-460), mammary cancer (MAXF 401NL, MCF-7, MDA-231 and MDA-468), melanoma (MEXF 276L, MEXF 394NL, MEXF 462NL, MEXF 514L and MEXF 520L), ovary cancer (OVXF 1619L, OVXF 899L and OVCAR-3), pancreatic cancer (PAXF 1657L and PANC-1), prostate cancer (22Rv1, DU-145, LNCaP and PC3M), pleuramesothelioma (PXF 1752L), renal cancer (RXF 1781 L, RXF 393NL, RXF 486L and RXF 944L), and uterine cancer (UXF 1138L) is added to a 96-well microtiter plate at a density of 3.6-11.2x10³ cells per well in RPMI-1640 medium containing 10% fetal calf serum and 0.1% gentamicin and incubated for 24 hours. After 24 hours RPMI-1640 medium containing 10% fetal calf serum and 0.1% gentamicin, together with a test compound, is added thereto. Then, this plate is incubated at 37°C for 4 days in a humidified atmosphere of air containing 5% CO₂. Thereafter, the cell culture medium with or without drug was replaced by 200 µl of an aqueous PI solution (7 µg/ml propidium iodide). Applying a freeze thaw cycle to permeabilize intact cell walls, staining of DNA was achieved, and the fluorescence of each well was measured using the Cy-tofluor 4000 microplate reader (excitation 530 nm, emission 620 nm), giving a direct relationship to the total cell number. Proliferation inhibition is expressed as test *ver*sus untreated control (T/C-value [%]). IC₅₀ and IC₇₀ values were determined by plotting compound concentration *versus* cell viability. The results thus obtained are shown in Table 1. Further analyses of compound B in a broad range of human tumor cell lines is shown in Table 2.

It can be seen from Table 1 that the griseusin derivatives A, B, E and F inhibit proliferation of tumor cell lines. Moreover, antitumor activity of the new derivatives B, E and F is much more pronounced as compared to the compound A and D, for which only antimicrobial activity has been described up to now.

Comparing antitumor activity of compound B in a collection of different tumor cell lines, obviously, the compound selectively inhibits the growth of distinct tumor types, such as mammary cancer, melanoma and renal cancer (cf. Table 2).

**Table 1. Proliferation Inhibition of Cancer Cells by griseusin derivatives A, B, D, E and F.**

| | IC₅₀ value [µg/mL] | | | | | |
|---|---|---|---|---|---|---|
| | GXF 251L | LXFL 529L | MAXF 401NL | MEXF 462NL | RXF 486L | UXF 1138L |
| A | 3.064 | 8.945 | 0.345 | 1.126 | 1.114 | 8.084 |
| B | 0.106 | 1.068 | 0.09 | 0.086 | 0.078 | 0.216 |
| D | n.d. | n.d. | n.d. | n.d. | n.d. | n.d. |
| E | 0.393 | 3.865 | 0.046 | 0.094 | 0.288 | 0.377 |
| F | 0.037 | 0.158 | 0.034 | 0.029 | 0.053 | 0.302 |

n.d., not detected in concentration range tested

**Table 2. Proliferation Inhibition of Cancer Cells by griseusin derivative B**

| Tumor Type | Cancer Cell Lines | IC50 µg/ml | IC70 µg/ml |
|---|---|---|---|
| Bladder | BXF 1218L | 0.056 | 0.133 |
| | T-24 | 0.507 | 1.137 |
| Glioblastoma | CNXF 498NL | 0.107 | 0.184 |
| Colon | HCT-116 | 0.697 | 1.333 |
| | HT-29 | 0.114 | 0.177 |
| Stomach | GXF 251 L | 0.106 | 0.178 |
| Head and Neck | HNXF 536L | 0.237 | 0.716 |
| Lung, non small cell | LXFA 1121 L | 0.083 | 0.147 |
| | LXFA 289L | 8.770 | 14.801 |
| | LXFA 526L | 0.129 | 0.206 |
| | LXFL 529L | 1.068 | 1.814 |
| | LXFA 629L | 0.234 | 0.671 |
| | H-460 | 0.490 | 1.042 |
| Breast | MAXF 401NL | 0.090 | 0.160 |
| | MCF-7 | 0.138 | 0.210 |
| | MDA-231 | 0.068 | 0.136 |
| | MDA-468 | 0.060 | 0.127 |
| Melanoma | MEXF 276L | 0.112 | 0.212 |
| | MEXF 394NL | 0.035 | 0.086 |
| | MEXF 462NL | 0.086 | 0.158 |
| | MEXF 514L | 0.087 | 0.149 |
| | MEXF 520L | 0.102 | 0.175 |
| Ovary | OVXF 1619L | 0.242 | 0.916 |
| | OVXF 899L | 1.265 | 2.249 |
| | OVCAR-3 | 0.521 | 1.309 |
| Pancreas | PAXF 1657L | 0.094 | 0.179 |
| | PANC-1 | 0.117 | 0.200 |
| Prostate | 22Rv1 | 0.071 | 0.139 |
| | DU-145 | 0.251 | 0.669 |
| | LNCaP | 0.107 | 0.175 |
| | PC3M | 0.402 | 0.929 |
| Pleuramesothelio | PXF 1752L | 0.124 | 0.208 |
| Kidney | RXF 1781 L | 0.407 | 1.134 |
| | RXF 393NL | 0.104 | 0.164 |
| | RXF 486L | 0.078 | 0.142 |
| | RXF 944L | 0.038 | 0.107 |
| Uterus | UXF 1138L | 0.216 | 0.524 |

Using various human cancer xenografts *in vitro,* the concentrations of compound B which inhibit formation of tumor colonies of tumor stem cells by 50% (IC₅₀) and 70% (IC₇₀) are determined in a clonogenic assay. Specifically, each solid tumor of bladder cancer (BXF 1036 and BXF 1299), cervical cancer (CEXF 633 and CEXF 773), colic cancer (CXF 1103, CXF 158, CXF 1729, CXF 264 and CXF 280), gastric cancer (GXF 251 and GXF 97), head and neck cancer (HNXF 536), liver cancer (LIXF 575), non small cell lung cancer comprising adeno, squamous epithelium and large cell carcinoma (LXFA 1012, LXFA 289, LXFA 297, LXFA 526, LXFA 592, LXFA 629, LXFA 677, LXFE 409, LXFL 1072, LXFL 430LX and LXFL 529), small cell lung cancer (LXFS 650), mammary cancer (MAXF 1162, MAXF 1384, MAXF 401, MAXF 449, MAXF 574, MAXF 583, MAXF 713 and MAXF 857), melanoma (MEXF 1341, MEXF 462, MEXF 535 and MEXF 989), ovary cancer (OVXF 899), pancreatic cancer (PAXF 546 and PAXF 736), prostate cancer (DU-145 and PC3M), pleuramesothelioma (PXF 1752), renal cancer (RXF 1220, RXF 1393, RXF 393, RXF 631 and RXF 944LX), and sarcoma (SXF 1301 and SXF 463) engrafted on RPMI nu/nu thymus aplastic nude mice were explanted, disaggregated by mincing and enzymatic digestion for 50 minutes using 125 U/mL DNAse I, 41 U/mL collagenase IV, 100 U/ml hyaluronidase III, and 1 U/mL dispase I, and passed through sieves of 200 µm and 50 µm mesh size and washed twice with sterile PBS-buffer to remove cell aggregated from single cells. The clonogenic assay was performed in a 24 multiwell format. Each of the resulting single cell suspensions was added at a density of 2·10⁴ to 6·10⁴ cells per well to 0.2 ml of Iscove's Modified Dulbecco's Medium (IMDM) containing 20% fetal calf serum and 0.01% gentamicin supplemented with 0.4% (w/v) agar, and plated onto a bottom layer consisting of the same culture medium containing 0.75% (w/v) agar. After 24 hours IMDM containing 20% fetal calf serum and 0.01 % gentamicin, together with a test compound, was added there to. Then, the plate was incubated at 37°C for 10 to 20 days in a humidified atmosphere of air containing 7.5% CO₂. Within this period, *in vitro* tumor growth led to the formation of colonies with a diameter of > 50µm. Colony counts were performed with an automatic image analysis system (OMNICON 3600). 24 hours prior to evaluation, vital colonies were stained with a vital staining dye. Therefore, 100 µl of a sterile aqueous solution of 2-(4-iodophenyl)-3-(4-nitrophenyl)-5-phenyltetrazolium chloride (1 mg/ml) were added to each well of the plate, and plates were incubated for 24 hours at 37°C in a humidified atmosphere of air containing 7.5% CO₂. Inhibition of colony formation was expressed as test versus untreated control (T/C-value [%]). IC₅₀ and IC₇₀ values were determined by plotting compound concentration versus cell viability. The results thus obtained are shown in Table 3.

**Table 3: Inhibition of Colony Formation of Tumor Stem Cells by griseusin derivative B**

| Tumor Type | Cancer Xenograft | IC50 µg/ml | IC70 µg/ml |
|---|---|---|---|
| Bladder | BXF 1036 | 1.743 | 2.271 |
| | BXF 1299 | 1.107 | 1.662 |
| Cervix | CEXF 633 | 1.310 | 1.837 |
| | CEXF 773 | 1.936 | 2.333 |
| Colon | CXF 1103 | 0.173 | 0.529 |
| | CXF 158 | 0.126 | 0.358 |
| | CXF 1729 | 0.453 | 0.630 |
| | CXF 264 | 0.103 | 0.215 |
| | CXF 280 | 0.379 | 0.609 |
| Stomach | GXF 251 | 0.333 | 0.677 |
| | GXF 97 | 0.300 | 0.495 |
| Head and Neck | HNXF 536 | 0.350 | 0.547 |
| Liver | LIXF 575 | 0.616 | 0.764 |
| Lung, non small cell | LXFA 1012 | 0.442 | 0.631 |
| | LXFA 289 | 0.218 | 0.425 |
| | LXFA 297 | 1.461 | 2.179 |
| | LXFA 526 | 0.300 | 0.512 |
| | LXFA 592 | 0.215 | 0.468 |
| | LXFA 629 | 0.808 | 1.276 |
| | LXFA 677 | 0.974 | 8.063 |
| | LXFE 409 | 1.732 | 2.249 |
| | LXFL 1072 | 0.252 | 0.468 |
| | LXFL 430LX | 0.272 | 0.468 |
| | LXFL 529 | 0.364 | 0.699 |
| Lung, small cell | LXFS 538 | 0.307 | 0.502 |
| Breast | MAXF 1162 | 0.182 | 0.362 |
| | MAXF 1384 | 0.606 | 0.750 |
| | MAXF 401 | 0.478 | 0.782 |
| | MAXF 449 | 0.400 | 0.648 |
| | MAXF 574 | 0.335 | 0.523 |
| | MAXF 583 | 0.078 | 0.195 |
| | MAXF 713 | 0.498 | 0.700 |
| | MAXF 857 | 1.449 | 1.975 |
| Melanoma | MEXF 1341 | 0.462 | 0.673 |
| | MEXF 462 | 1.666 | 2.152 |
| | MEXF 535 | 0.055 | 0.094 |
| | MEXF 989 | 0.118 | 0.182 |
| Ovary | OVXF 899 | 0.468 | 0.731 |
| Pancreas | PAXF 546 | 0.168 | 0.420 |
| | PAXF 736 | 0.246 | 0.452 |
| Prostate | DU-145 | 1.086 | 1.644 |
| | PC3M | 0.419 | 0.818 |
| Pleuramesothelio | PXF 1752 | 0.811 | 1.253 |
| Kidney | RXF 1220 | 0.782 | 0.952 |
| | RXF 1393 | 0.592 | 0.769 |
| | RXF 393 | 0.150 | 0.212 |
| | RXF 631 | 0.517 | 0.719 |
| | RXF 944LX | 0.116 | 0.171 |
| Sarcoma | SXF 1301 | 0.328 | 0.517 |
| | SXF 463 | 0.235 | 0.334 |

It can be seen from Table 3 that griseusin derivative B inhibits colony formation of tumor stem cells derived from solid tumor xenografts. Comparing antitumor activity of compound B in a large collection of different tumor xenografts, obviously, the compound selectively inhibits the growth of distinct tumor types, such as colon cancer, mammary cancer, melanoma and renal cancer (cf. Table 3).

The present invention provides new griseusin derivatives which strongly inhibit the growth of cultured tumor cells. Accordingly, the present invention can be utilized in the fields of pharmaceutics and medicine.

## Claims

1. A griseusin derivative represented by the following formula (I): 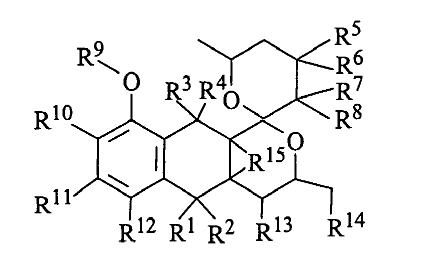 wherein R¹, R², R³ and R⁴ independently from each other represent hydrogen, hydroxy, a straight-chain or branched-chain alkyl group having 1 to 12 carbon atoms, a straight-chain or branched-chain alkoxy group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a substituted or unsubstituted aryloxy group which can comprise one or more heteroatoms, a (-C(O)-Rₐ) group or a (-C(S)-Rₐ) group, or
at least one of R¹ and R² when taken together and R³ and R⁴ when taken together, respectively, stands for an oxo group (=O), thioxo group (=S), imino group (=NRₐ), cyanimino group (=NCN) or alkylidene group (=CRₐR_{b}),
with Rₐ and R_{b} independently from each other being selected from a group α, consisting of hydrogen, a straight-chain or branched-chain alkyl group having 1 to 12 carbon atoms, a cycloalkyl group having 3 to 10 carbon atoms and optionally comprising one or more heteroatoms, and a substituted or unsubstituted aryl group which can comprise one or more heteroatoms, a substituted or unsubstituted aralkyl group which can comprise one or more heteroatoms, or
at least one of R¹ and R² when taken together and R³ and R⁴ when taken together, respectively, forms together with the respective spiro carbon atom of ring B a 5 to 8 membered cyclic ketal structure and being optionally substituted by one or more substituents selected from group α, or
R², R⁴ and R¹⁵ are not present so that ring B is an aromatic ring substituted in its para-positions by R¹ and R³;
R⁵, R⁶, R⁷ and R⁸ independently from each other represent hydrogen, hydroxy, a straight-chain or branched-chain alkyl group having 1 to 12 carbon atoms, a straight-chain or branched-chain alkoxy group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a substituted or unsubstituted aryloxy group which can comprise one or more heteroatoms, a (-C(O)-Rₐ) group, a (-C(S)-Rₐ) group, a (-OC(O)-Rₐ) group, a (-OC(S)-Rₐ) group, a (-OC(O)-ORₐ) group or a (-(CH₂)ₙ-CRₐ=CRₐR_{b}) group wherein n is zero or an integer from 1 to 12 and with Rₐ and R_{b} being as defined above, or
at least one of R⁵ and R⁶ when taken together and R⁷ and R⁸ when taken together, respectively, stands for an oxo group (=O), thioxo group (=S), imino group (=NRₐ), cyanimino group (=NCN) or alkylidene group (=CRₐR_{b}) with Rₐ and R_{b} as defined above, or forms together with the respective spiro carbon atom a cyclic ketal structure having 5 to 8 carbon atoms and optionally substituted by one or more substituents selected from group α;
R⁹ represents hydrogen, a straight-chain or branched-chain alkyl group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a (-C(O)-Rₐ) group, a (-C(S)-Rₐ) group or a (-(CH₂)ₙ-CRₐ=CRₐR_{b}) group with n, Rₐ and R_{b} as defined above;
R¹⁰, R¹¹ and R¹² are independently glycosidic groups particularly of furanoic or pyranoic structure and optionally being deoxygenated or aza-substituted;
R¹³ represents hydrogen, hydroxy, a straight-chain or branched-chain alkoxy group having 1 to 12 carbon atoms, a (-C(O)-Rₐ) group, a (-C(S)-Rₐ) group, a (-OC(O)-Rₐ) group or a (-OC(S)-Rₐ) group, with Rₐ as defined above,
R¹⁴ represents hydrogen, hydroxy, a straight-chain or branched-chain alkyl group having 1 to 12 carbon atoms, a straight-chain or branched-chain alkoxy group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a substituted or unsubstituted aryloxy group which can comprise one or more heteroatoms, a (-C(O)-Rₐ) group, a (-C(S)-Rₐ) group, a (-OC(O)-Rₐ) group, a (-OC(S)-Rₐ) group or a (-COORₐ) group with Rₐ as defined above;
or R¹³ and R¹⁴ when taken together form the following lacton structure or lactol structure, respectively, 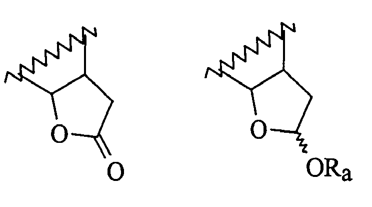 with Rₐ as defined above, and
R¹⁵ represents an alkylene group (-C(Rₐ)(R_{b})-), a (-CH₂-N=N-) group, an oxygen atom or a sulfur atom with Rₐ and R_{b} as defined above; or
R¹⁵ is not present, but stands for a double bond together with the adjacent ring carbon atoms in ring B to form a quinoid or semi-quinoid system in said ring B;
with the proviso that the griseusin derivative is not spiro[1H-naphtho[2,3-c]pyran-1,2'-[2H]pyran]-3-acetic acid, (3,3',4,4',5,5',6',10-octahydro-3',4',9-trihydroxy-6'-methyl-5,10-dioxo-), spiro[1H-naphtho[2,3-c]pyran-1,2'-[2H]pyran]-3-acetic acid, (4'-(acetyloxy)-3,3',4,4',5,5',6',10-octahydro-3',9-dihydroxy-6'-methyl-5,10-dioxo-), spiro[1H-naphtho[2,3-c]pyran-1,2'-[2H]pyran]-3-acetic acid, (3',4'-bis(acetyloxy)-3,3',4,4',5,5',6',10-octahydro-9-hydroxy-6'-methyl-5,10-dioxo-), spiro[5H-furo[3,2-b]naphtho[2,3-d]pyran-5,2'-[2H]pyran]-2,6,11(3H)-trione, (3',3a,4',5',6',11b-hexahydro-3',4',7-trihydroxy-6'-methyl-), spiro[5H-furo[3,2-b]naphthol[2,3-d]pyran-5,2'-[2H]pyran]-2,6,11(3H)-trione, (4'-(acetyloxy)-3',3a,4',5',6',11b-hexahydro-3',7-dihydroxy-6'-methyl-), spiro[5H-furo[3,2-b]naphtho[2,3-d]pyran-5,2'-[2H]pyran]-2,6,11(3H)-trione,3'4'-bis(acetyloxy)-3',3a,4',5',6',11b-hexahydro-7-hydroxy-6'-methyl-, spiro[1H-naphtho[2,3-c]pyran-1,2'-[2H]pyran]-3-acetic acid, 4'-(acetyloxy)-3,3',4,4',5,5',6',10-octahydro-3'hydroxy-9-methoxy-6'-methyl-5,10-dioxo-, spiro[5H-furo[3,2-b]naphtho[2,3-d]pyran-5,2'-[2H]pyran]-2,6,11(3H)-trione, 4'-(acetyloxy)-3',3a,4',5',6',11b-hexahydro-7-methoxy-6'-methyl-3'-(phenylmethoxy)-, spiro[5H-furo[3,2-b]naphtho[2,3-d]pyran-5,2'-[2H]pyran]2,6,11(3H)-trione, 3',4',7-tris(acetyloxy)-3',3a,4',5',6',11b-hexahydro-6'-methyl-carbonic acid, 4'-(acetyloxy)-2,3,3',3a,4',5',6,6',11,11b-decahydro-7-hydroxy-6'-methyl-2,6,11-trioxospiro[5H-furo[3,2-b]naphtho[2,3-d]pyran-5,2'-[2H]pyran]-3'-yl methyl ester, and carbonic acid, 4'-(acetyloxy)-2,3,3',3a,4',5',6,6',11,11b-decahydro-6'methyl-2,6,11-trioxospiro[5H-furo[3,2-b]naphtho[2,3-d]pyran-5,2'-[2H]pyran]-3',7-diyl dimethyl ester.

2. The griseusin derivative according to claim 1, wherein
R¹, R², R³ and R⁴ independently from each other represent hydrogen, hydroxy, a straight-chain or branched-chain alkoxy group having 1 to 12 carbon atoms or
R¹/R² and/or R³/R⁴ stand(s) for an oxo group (=O) or form(s) together with the respective spiro carbon atom of ring B a cyclic ketal structure having 5 to 8 carbon atoms and optionally substituted by one or more substituents selected from group α;
R⁵, R⁶, R⁷ and R⁸ independently from each other represent hydrogen, hydroxy, a straight-chain or branched-chain alkyl group having 1 to 12 carbon atoms, a straight-chain or branched-chain alkoxy group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a substituted or unsubstituted aryloxy group which can comprise one or more heteroatoms, a (-C(O)-Rₐ) group, a (-C(S)-Rₐ) group, a (-OC(O)-Rₐ) group, a (-OC(S)-Rₐ) group, a (-OC(O)-ORₐ) group or a (-(CH₂)ₙ-CRₐ=CRₐR_{b}) group with n, Rₐ and R_{b} being as defined above, or
R⁵/R⁶ and/or R⁷/R⁸ stand(s) for an oxo group (=O) or form(s) together with the respective spiro carbon atom of ring B a 5 to 8 membered cyclic ketal structure being optionally substituted by one or more substituents selected from group α,
R⁹ represents hydrogen, a straight-chain or branched-chain alkyl group having 1 to 12 carbon atoms, a cycloalkyl group having 3 to 10 carbon atoms and optionally comprising one or more heteroatoms, a substituted or unsubstituted aryl group which can comprise one or more heteroatoms, a substituted or unsubstituted aralkyl group which can comprise one or more heteroatoms, a (-C(O)-Rₐ) group or a (-C(S)-Rₐ) group with Rₐ as defined above;
R¹³ represents hydrogen, hydroxy, a straight-chain or branched-chain alkyl group having 1 to 12 carbon atoms, a straight-chain or branched-chain alkoxy group having 1 to 12 carbon atoms or a (-C(O)-Rₐ) group,
R¹⁴ represents hydroxy, a straight-chain or branched-chain alkoxy group having 1 to 12 carbon atoms, a (-OC(O)-Rₐ) group, a (-OC(S)-Rₐ) group or a (-COORₐ) group with Rₐ as defined above,
or R¹³ and R¹⁴ when taken together form said lacton structure 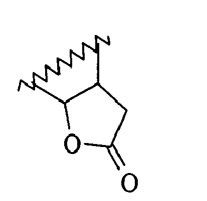 and
R¹⁵ represents an oxygen atom or is not present, but stands for a double bond together with the adjacent ring carbon atoms in ring B.

3. The griseusin derivative according to claim 1, wherein
the groups R¹/R² and R³/R⁴ each form a carbonyl group;
at least one of R⁵/R⁶ and R⁷/R⁸ forms a carbonyl group, while the remaining R⁵ and R⁶ or R⁷ and R⁸, respectively, independently from each other represent hydrogen, hydroxy, a (-OC(O)-Rₐ) group or a (-OC(S)-Rₐ) group with Rₐ as defined above, or both of R⁵/R⁶ and R⁷/R⁸ form a carbonyl group;
R⁹ represents hydrogen or a straight-chain or branched-chain alkyl group having 1 to 12 carbon atoms;
R¹³ represents hydroxy and R¹⁴ represents a (-COORₐ) group with Rₐ as defined above or R¹³ and R¹⁴ when taken together form said lacton structure 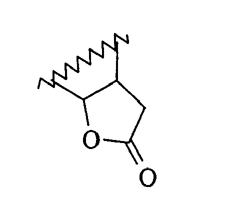 and
R¹⁵ is not present, but stands for a double bond together with the adjacent ring carbon atoms in ring B.

4. A pharmaceutical composition comprising a griseusin derivative according to any one of claims 1 to 3 together with a pharmaceutically acceptable carrier or diluent.

5. Use of a griseusine derivative as defined in any one of claims 1 to 3 for the manufacture of a medicament for inhibiting cancer.

6. Use according to claim 5, wherein the cancer is a human solid tumor and metastasis thereof.

7. Use according to claim 5 or 6, wherein the human solid tumor is selected from the group consisting of sarcomas; bronchogenic carcinoma, alveolar carcinoma and mesothelioma of the lung; squamous cell carcinoma and adenocarcinoma of the esophagus; carcinoma and leiomyosarcoma of the stomach; ductal adenocarcinoma of the pancreas; adenocarcinoma, tubular adenoma, villous adenoma, hamartoma and leiomyoma of the large bowel; adenocarcinoma of the kidney; squamous cell carcinoma, transitional cell carcinoma and adenocarcinoma of the bladder and urethra; adenocarcinoma of the prostate; seminoma, teratoma, embryonal carcinoma, teratocarcinoma and choriocarcinoma of the testis; hepatoma, cholangiocarcinoma and hepatoblastoma of the liver; osteogenic sarcoma, fibrosarcoma and malignant fibrous histiocytoma of the bone; astrocytoma, medulloblastoma, glioma, ependymoma, glioblastoma multiform and oligodendroglioma of the brain; endometrial carcinoma of the uterus; cervical carcinoma of the cervix; ovarian carcinoma, granulosa-theca cell tumors of the ovaries; squamous cell carcinoma, intraepithelial carcinoma, adenocarcinoma, fibrosarcoma and melanoma of the vulva; malignant melanoma, basal cell carcinoma and squamous cell carcinoma of the skin.

8. Use according to anyone of claims 5 to 7, wherein the griseusin derivative is in the form of a physiologically acceptable salt.
